# EUROPÄISCHE PATENTSCHRIFT

(11) **EP 3 870 251 B1**
(45) Veröffentlichungstag und Bekanntmachung des Hinweises auf die Patenterteilung: **16.04.2025**
(21) Anmeldenummer: 19795141.1
(22) Anmeldetag: 22.10.2019
(51) Int. Cl.: A61M 1/28

(54) **DIALYSEGERÄT FÜR DIE PERITONEALDIALYSE**
DIALYSIS APPARATUS FOR PERITONEAL DIALYSIS
APPAREIL DE DIALYSE POUR LA DIALYSE PÉRITONÉALE

(30) Priorität: 26.10.2018 DE 102018126817
(43) Veröffentlichungstag der Anmeldung: 01.09.2021
(73) Patentinhaber: Fresenius Medical Care Deutschland GmbH, 61352 Bad Homburg (DE)
(72) Erfinder: GRIESSMANN, Erik, 97422 Schweinfurt (DE); RATHKE-SCHLAEFER, Bettina, 61250 Usingen (DE)
(74) Vertreter: Herrmann, Uwe
(86) Internationale Anmeldenummer: PCT/EP2019/078662
(87) Internationale Veröffentlichungsnummer: WO 2020/083877

(56) Entgegenhaltungen:
- DE-A1- 102011 105 916
- US-A1- 2012 071 815
- US-B1- 6 558 343

## Beschreibung

Die Erfindung betrifft ein Dialysegerät zur Durchführung einer automatisierten Peritonealdialyse-(APD)-Behandlung.

In der Durchführung von automatisierten Peritonealdialyse-Behandlungen ist es aus der WO 2013/000569 A1 bekannt, die Behandlung unter Einhaltung vorgegebener Toleranzen und zeitlicher Hinsicht sowie unter dem Aspekt des verabreichten Volumens an Dialyseflüssigkeit zu optimieren. Wesentlich sind im Rahmen dieses Konzepts die Definitionen einer zulässigen Verkürzung der Verweildauer, einer zulässigen Reduzierung des Einlaufvolumens, eines zulässigen Restvolumens und eines zulässigen Patientenvolumens.

Keine Berücksichtigung findet die potentielle Verwendung bzw. Verabreichung nicht verwendeter Dialyselösung. Ist am Ende der Behandlung noch frische Dialyseflüssigkeit übrig, wird diese beim Entleeren der Lösungsbeutel in die Drainage gefördert. Jedoch kommt es in der Praxis häufig vor, dass das verschriebene Gesamt-Einlaufvolumen einer Behandlung dem Patienten nicht vollständig verabreicht werden kann. Die Ursache hierfür liegt insbesondere in den unterschiedlichen Wertebereichen für das zulässige Restvolumen und das zulässige Patientenvolumen.

Das zulässige Restvolumen wird im Verhältnis zum zulässigen Patientenvolumen typischerweise relativ hoch eingestellt, damit der Patient nicht durch Auslauf-Störungsmeldungen im Schlaf gestört wird.

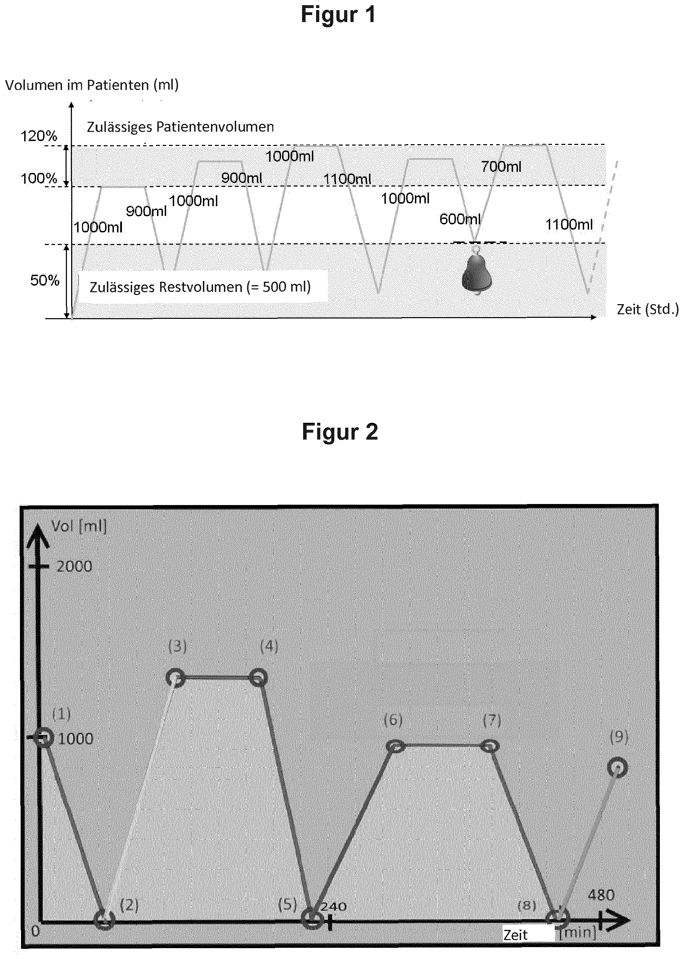

Dieser Sachverhalt ist schematisch in Figur 1 erklärt. Das zulässige Patientenvolumen beträgt im dort dargestellten Beispiel 120%, während das zulässige Restvolumen auf 50% festgelegt ist. Das verschriebene Gesamt-Einlaufvolumen beträgt 5000 ml (5 Zyklen à 1000 ml). Aufgrund der automatischen Umschaltung im Residual-Bereich kann aber nur 4700 ml Lösung verabreicht werden. Während nämlich bei Erreichen von 50% des Auslaufvolumens bereits in den nächsten Einlauf geschalten werden darf, kann wegen der oberen Grenze für das zulässige Einlaufvolumen nur 20% mehr an Einlaufvolumen verabreicht werden. So bleibt Dialyselösung ungenutzt, was einerseits zu inadäquaten Behandlungsergebnissen führen kann und andererseits auch unwirtschaftlich ist.

Um einen Verwurf der Restflüssigkeit, also der Differenz aus angeschlossenem Dialysat-Volumen und dem für die Behandlung tatsächlich benötigten Volumen zu vermeiden, wäre die Verschreibung einer Tidal-Behandlung denkbar, wobei ein Tidal-Auslauf volumengesteuert verabreicht wird. Dadurch kann der Verlust an Dialyselösung durch das vorgegebene zulässige Restvolumen minimiert werden. Da der Patient aber nicht soweit entleert werden kann wie durch einen druckgesteuerten Auslauf, entsprechend der Standard-Behandlung, wird die Effizienz der Behandlung minimiert. Die Tidal-Behandlung wird deswegen häufig nicht aus therapeutischen Gründen verschrieben, sondern lediglich um Störungen und Verlust von Dialyseflüssigkeit zu minimieren. In diesem Zusammenhang ist das in der WO 2010/088360 A1 beschriebene Verfahren zu nennen, wobei im Falle der Erkennung eines unvollständigen Auslaufs die restliche Behandlung durch eine Tidal-Behandlung ersetzt wird, um das verschriebene Gesamtvolumen umzusetzen um eine Ziel-UF-Rate zur erreichen. Das verschriebene Rest-Einlaufvolumen wird dabei gleichmäßig so auf die Tidal-Zyklen verteilt, sodass die Gesamtbehandlungszeit eingehalten wird.

Die US 6 558 343 B1 offenbart ebenso ein Verfahren einer Tidal-Behandlung.

Ferner wäre denkbar, die Verschreibung während der Behandlung zu editieren. Wird im Laufe der Behandlung häufig an der Grenze des zulässigen Restvolumens umgeschaltet, um die nächste Inflow-Phase zu initiieren, bestünde die Möglichkeit, dass der Benutzer mit einem Editor die nachfolgenden Volumina der Einläufe erhöht. Die Möglichkeit zu Editieren ist aber an Benutzerrechte gebunden, der Benutzer müsste nachts einen Wecker stellen und die Anpassung ist relativ aufwändig.

Weiterhin wäre denkbar, die Grenze für das zulässige Restvolumen herabzusetzen. Dies kann allerdings dazu führen, dass der Patient während der Behandlung auf Grund von Störungen häufiger geweckt wird. Der Parameterwert für das zulässige Restvolumen wird ja regelmäßig genau deshalb hoch eingestellt, um das zu vermeiden.

Aufgrund der genannten Nachteile führt allerdings keines dieser Konzepte zu einem wirklich zufriedenstellenden Konzept. Daneben wäre die Implementierung dieser Konzepte in Geräte problematisch, die profilierte Verschreibungen modellieren, wobei dem Patienten in jedem Zyklus ein individuelles Volumen einer individuell angepassten Dialyselösung verabreicht werden kann, wie in Figur 2 schematisch dargestellt.

Aufgabe der Erfindung ist es, ein verbessertes Konzept zur Nutzung der Restflüssigkeit bereitzustellen.

Vor diesem Hintergrund betrifft die Erfindung eine Vorrichtung zur Durchführung einer Peritonealdialyse-Behandlung, umfassend ein Reservoir für frische Dialyseflüssigkeit, einen Anschluss zur Verbindung mit einem Peritoneal-Katheter eines Patienten, einen Ablauf für verbrauchte Dialyseflüssigkeit und eine mit Aktoren in Verbindung stehende Steuereinheit, wobei die Vorrichtung ausgebildet ist, auf der Grundlage einer in der Steuereinheit hinterlegten Verschreibung für eine Behandlung mehrere aufeinanderfolgende Inflow-Dwell-Outflow-Zyklen durchzuführen, in welchen dem Patienten Dialyseflüssigkeit aus dem Reservoir zugeführt und nach Ablauf einer bestimmten Verweilzeit im Peritoneum des Patienten wieder durch den Ablauf abgeleitet wird, und wobei die Vorrichtung ferner ausgebildet ist, in wenigstens einem Zyklus einen Teil der im Peritoneum des Patienten befindlichen Dialyseflüssigkeit bereits vor Ablauf der Verweilzeit mit frischer Dialyseflüssigkeit aus dem Reservoir zu ersetzen, wenn in wenigstens einem vorhergehenden Zyklus das gemäß Verschreibung für diesen vorhergehenden Zyklus vorgesehene Volumen an frischer Dialyseflüssigkeit nicht vollständig verbraucht wurde.

Gemäß der Erfindung ist also vorgesehen, gemäß Verschreibung voraussichtlich nicht mehr gebrauchte frische Dialyselösung für eine Auffrischung der im Peritoneum des Patienten befindlichen Dialyselösung während der Verweilphase zu verwenden.

Im Rahmen des vorzeitigen Teilersatzes können in einer Ausführungsform maximal 50%, vorzugsweise maximal 30% und weiter vorzugsweise maximal 20% des insgesamt im Peritoneum befindlichen Volumens an Dialyseflüssigkeit ersetzt werden. Es geht im Rahmen der Erfindung also nicht um den faktischen Einbau weiterer vollständiger Ab- und Einlaufphasen, sondern vielmehr um den Ersatz lediglich einer kleineren Teilmenge der im Peritoneum befindlichen Dialyseflüssigkeit, um diese etwas aufzufrischen.

Vorzugsweise wird die Verweilzeit, also die Dauer der Dwell-Phase des jeweiligen Zyklus durch den vorzeitigen Teilersatz nicht verlängert oder verkürzt. Die Verweilzeit bleibt also konstant. Wenn in diesem Zusammenhang von Verweilzeit die Rede ist, ist die Verweilzeit einschließlich des Teilersatzvorgangs gemeint. So bleibt die gesamte Behandlungsdauer unverändert.

In dieser Ausführungsform wird das im Peritoneum des Patienten befindliche Gesamtvolumen an Dialyseflüssigkeit durch den vorzeitigen Teilersatz nicht verändert. Es ist also vorgesehen, dass das im Peritoneum des Patienten befindliche Gesamtvolumen an Dialyseflüssigkeit nach dem Teilersatzvorgang dem im Peritoneum des Patienten befindlichen Gesamtvolumen an Dialyseflüssigkeit vor dem Teilersatzvorgang entspricht. Während des Teilersatzvorgangs ergeben sich kleine Abweichungen.

Alternativ kann auch vorgesehen sein, dass sich das Volumen geringfügig verändert, beispielsweise insbesondere dann, wenn der Teilersatzvorgang mit einem Zulaufschritt von frischer Dialyselösung beginnt.

Der Teilersatzvorgang kann sowohl mit einem Zulaufschritt als auch mit einem Ablaufschritt beginnen, wobei der Beginn mit einem Ablaufschritt bevorzugt sein kann, insbesondere wenn das im Peritoneum des Patienten befindliche Gesamtvolumen nahe an einem festgelegten dem Maximalvolumen liegt. Andererseits können bei einem Beginn mit einem Zulaufschritt eventuelle Ablaufprobleme verringert werden, da der Ablaufdruck dann höher wäre.

Vorzugsweise erfolgt der vorzeitige Teilersatz in mehreren vorzugsweise unmittelbar aufeinanderfolgenden Zu- und Ablaufschritten. Durch die Aufteilung des zum Teilersatz notwendigen Ab- und Zulaufs von Dialyseflüssigkeit in mehrere Schritte kann das Gesamtvolumen der Flüssigkeit im Peritoneum annähernd konstant gehalten werden. Mehrere kleinere Teilersatzvolumina minimieren auch das Risiko von Auslaufproblemen. Wenn trotzdem ein problematisches Aus- oder Einlaufereignis auftritt, kann das Teilersatzverfahren abgebrochen und gegebenenfalls zu einem späteren Zeitpunkt fortgeführt werden. Alternativ kann beim Auftreten eines Auslaufproblems versucht werden, den Teilersatzzyklus mit einem Einlauf fortsetzten, um einen eventuell festgesaugten Katheter freispülen zu können.

In diesem Zusammenhang können beispielsweise drei oder mehr, fünf oder mehr oder dergleichen Anzahlen an Zulaufschritten vorgesehen sein und/oder mit jedem Zulaufschritt maximal 200 ml zugeführt werden. Durch eine hohe Zahl an kleinvolumigen Zu- und Ablaufschritten während des Teilersatzvorgangs kann das effektive Teilersatzvolumen erhöht werden.

Vorzugsweise ist vorgesehen, dass der vorzeitige Ersatz erst nach Ablauf von wenigstens 50% der vorgesehenen Verweilzeit durchgeführt wird und/oder dass der vorzeitige Ersatz vor Ablauf von maximal 80% der vorgesehenen Verweilzeit beendet ist. Nach Ablauf der halben Verweilzeit ist die im Peritoneum befindliche Dialyseflüssigkeit regelmäßig nicht mehr so wirksam wie zu Beginn der Verweilzeit, sodass durch den Teilersatz eine merkliche Effizienzsteigerung erreicht werden kann. Ein zu später Ersatz hat nur geringe Auswirkungen auf die Qualität der Behandlung, da die aufgefrischte Lösung ihre Wirkung dann nicht mehr in spürbarem Umfang entfalten kann.

In einer Ausführungsform ist die Vorrichtung ausgebildet, über den Verlauf der Behandlung in mehreren Zyklen einen Teil der im Peritoneum des Patienten befindlichen Dialyseflüssigkeit bereits vor Ablauf der Verweilzeit mit frischer Dialyseflüssigkeit aus dem Reservoir zu ersetzen. Dies hat einerseits Effizienzgründe, andererseits kann sich die Situation bezüglich vorhandenen Restvolumen aber auch im Laufe der Behandlung ändern. So muss beispielsweise gegebenenfalls Dialyselösung verworfen werden, wenn Störungen auftreten oder Pumpen neu positioniert werden.

In einer Ausführungsform ist die Vorrichtung ausgebildet, einen Teilersatzzyklus nur dann zu starten, wenn die gemäß Verschreibung für den jeweiligen Zyklus vorgesehene Verweilzeit eine Mindestzeit überschreitet. Für Zyklen mit kurzen Verweilzeiten macht es auf Grund der kurzen Einwirkzeit gegebenenfalls keinen Sinn einen Teilersatzzyklus zu generieren. Geeignete Mindestzeiten können beispielsweise auf 45 Minuten oder 60 Minuten festgelegt sein.

Die Festlegung, ob ein Teilersatz stattfindet, wird von der Vorrichtung typischerweise zu Beginn einer Verweilphase getroffen, da zu diesem Zeitpunkt das verfügbare und benötigte Einlaufvolumen ermittelt werden kann. Weiterhin kann die Verweilphase modifiziert werden. Beispielsweise kann die vorgegebene Verweilzeit verkürzt werden. Anschließend werden ein oder mehrere Aus- und Einläufe mit geringen Volumen verabreicht. Nach dem Teilersatz folgt eine weitere abschließende Verweilphase. Der geplante Zeitpunkt für den nachfolgenden Auslauf soll weiterhin eingehalten werden, damit es nicht zu einer Behandlungsverlängerung kommt.

Im Rahmen des Teilersatzes könnte man grundsätzlich auch in Erwägung ziehen, je nach Verfügbarkeit eine Lösung mit einer anderen Glukosekonzentration zu verabreichen, als für den Zyklus ursprünglich vorgesehen war, da sich aufgrund der relativ geringen Mengen die Gesamtkonzentration nur geringfügig ändern würde.

Als zusätzlicher Effekt ist denkbar, dass sich aufgrund der Tatsache, dass die Dialyselösung durch den Teilersatz im Peritoneum umgewälzt wird, ein zusätzlicher positiver Effekt bei der Behandlungseffizienz erzielt wird.

Die Zufluss- und Ablaufraten während des Teilersatzzyklus können generell entsprechend der Raten für Inflow und Outflow des Zyklus festgelegt werden oder aber geringer oder größer sein. In diesem Zusammenhang können auch sehr geringe Flussraten vorgesehen werden, um einen quasi kontinuierlichen Teilersatz vorzunehmen.

Gegebenenfalls müssen etwaige Minimalvolumina, die für eine Verschreibung gelten, für die Wahl des Teilersatzvolumens nicht beachtet werden, da es sich bei dem Teilersatz um keinen Verschreibungsparameter handelt. Vielmehr würde ein entsprechender Patientenparameter übergreifend für alle Verschreibungen gelten.

Die Erfindung betrifft weiterhin eine Vorrichtung zur Durchführung einer Peritonealdialyse-Behandlung, umfassend ein Reservoir für frische Dialyseflüssigkeit, einen Anschluss zur Verbindung mit einem Peritoneal-Katheter eines Patienten, einen Ablauf für verbrauchte Dialyseflüssigkeit und eine mit Aktoren in Verbindung stehende Steuereinheit, wobei die Vorrichtung ausgebildet ist, auf der Grundlage einer in der Steuereinheit hinterlegten Verschreibung für eine Behandlung mehrere aufeinanderfolgende Zyklen, typischerweise Inflow-Dwell-Outflow-Zyklen durchzuführen, in welchen dem Patienten Dialyseflüssigkeit aus dem Reservoir zugeführt und nach Ablauf einer bestimmten Verweilzeit im Peritoneum des Patienten wieder durch den Ablauf abgeleitet wird, und wobei die Vorrichtung ferner ausgebildet ist, in wenigstens einem Zyklus das gesamte Einlaufvolumen zu erhöhen, wenn in wenigstens einem vorhergehenden Zyklus das gemäß Verschreibung für diesen vorhergehenden Zyklus vorgesehene Volumen an frischer Dialyseflüssigkeit nicht vollständig verbraucht wurde.

Gemäß dieser Variante der Erfindung ist also vorgesehen, gemäß Verschreibung voraussichtlich nicht mehr gebrauchte frische Dialyselösung für eine Aufstockung eines späteren Zyklus zu verwenden. Diese Variante der Verfahrensführung könnte für adaptierte Verschreibungen vorteilhaft sein, da sich in dieser Behandlungsform Zyklen mit kleinen und großen Einlaufvolumina abwechseln. Die Zyklen mit den kleinen Einlaufvolumina könnten innerhalb vorgegebener Grenzen von beispielsweise 10% angehoben werden.

Die beiden Varianten der erfindungsgemäßen Verfahrensführung können kombiniert werden.

Generell kann vorgesehen sein, dass in der Steuereinheit ein Maximalvolumen an Dialyseflüssigkeit festgelegt ist, das sich im Peritoneum des Patienten befinden darf, und dass der nicht vollständige Verbrauch des für den vorhergehenden Zyklus vorgesehenen Volumens an frischer Dialyseflüssigkeit daraus resultiert, dass der vollständige Verbrauch zu einer Überschreitung des Maximalvolumens geführt hätte. Die Festlegung des Maximalvolumens dient der Patientensicherheit. Die Verschreibung ist grundsätzlich natürlich so ausgelegt, dass das Maximalvolumen nicht überschritten wird, wobei aber nicht immer in ausreichendem Maße berücksichtigt werden kann, dass im Rahmen der vorgelagerten Ablaufphase das Peritoneum nur unvollständig entleert wurde und sich das in der Verschreibung festgelegte Einlaufvolumen mit dem nicht prognostizierbaren Restvolumen zu einem Gesamtvolumen aufaddiert, welches dann potentiell über dem Maximalwert liegen kann. Das Restvolumen resultiert daraus, dass der Ablauf insbesondere während der Nachtruhe des Patienten nicht immer unter idealen Bedingungen erfolgen kann, dass aber ein gewisses Restvolumen toleriert wird, damit der Patient nicht durch Auslauf-Störungsmeldungen im Schlaf gestört wird.

In einer Ausführungsform ist die Vorrichtung ausgebildet, dem Patienten im Verlauf der Behandlung die gemäß der Verschreibung vorgesehene Gesamtmenge der Dialyseflüssigkeit zuzuführen, wobei eine etwaige Differenzmenge, die nicht im Rahmen der regulären Einlaufphasen zugeführt wird, insbesondere aufgrund der Berücksichtigung des Maximalvolumens, vollständig im Rahmen des vorzeitigen Ersatzes während des wenigstens einen oder mehrerer Zyklen zugeführt wird. So wird die vorhandene bzw. gemäß Verschreibung zur Verabreichung bestimmte Dialyselösung insgesamt sinnvoll eingesetzt, was sowohl aus medizinischer als auch aus ökonomischer Sicht wünschenswert ist.

Weitere Einzelheiten und Vorteile der Erfindung ergeben sich aus den nachfolgend anhand der Figuren beschriebenen Versuchen und Ausführungsbeispielen. In den Figuren zeigen:
- Figur 1:: eine schematische Darstellung eines Ablaufs einer automatisierten Peritonealdialyse-Behandlung, wobei das zulässige Restvolumen im Verhältnis zum zulässigen Patientenvolumen relativ hoch eingestellt ist und deshalb die Dialyselösung nicht vollständig verbraucht wird;
- Figur 2:: eine schematische Darstellung eines Ablaufs einer automatisierten Peritonealdialyse-Behandlung mit profilierter Verschreibung, wobei für den Einlauf 1 die Lösung A, für den Einlauf 2 die Lösung B und für den letzten Einlauf die Lösung C verschrieben wird (profilierte Lösungen) und wobei auch die Einlaufvolumina und Verweilzeiten für die beiden Zyklen variieren (profilierte Volumina und profilierte Verweilzeiten);
- Figur 3:: schematische Darstellung des Aufeinandertreffens von im Peritoneum des Patienten vorhandener Residual-Lösung und frischer Lösung während des Einlaufs;
- Figur 4:: schematische Darstellung der Vermischung der Residual-Lösung und der frischen Lösung;
- Figur 5:: eine schematische Darstellung einer Resorptionskurve für einen währende einer Peritonealdialyse-Behandlung ausgetauschten Soluten;
- Figur 6:: verschiedene Resorptionskurven für unterschiedliche Soluten;
- Figur 7:: ein Füllvolumen-Zeit-Diagramm für eine Verfahrensführung bei einer Peritonealdialyse-Behandlung anhand eines erfindungsgemäßen Geräts;
- Figur 8:: eine Darstellung, welche die Effektivität der UF-Generierung für Lösungen mit Glukosekonzentrationen von 3,86%, 2,27% und 1,50% zeigt;
- Figur 9:: Veranschaulichung der Effektivität einer Mischlösung aus frischer und gebrauchter Dialyselösung;
- Figur 10:: ein Füllvolumen-Zeit-Diagramm für eine alternative Verfahrensführung bei einer Peritonealdialyse-Behandlung anhand eines erfindungsgemäßen Geräts;
- Figur 11:: eine beispielhafte Darstellung von Anteilen des Teilersatzvolumens im Peritoneum;
- Figur 12:: ein Füllvolumen-Zeit-Diagramm für eine weitere Verfahrensführung bei einer Peritonealdialyse-Behandlung anhand eines erfindungsgemäßen Geräts; und
- Figur 13:: ein Füllvolumen-Zeit-Diagramm für eine wiederum andere Verfahrensführung bei einer Peritonealdialyse-Behandlung anhand eines erfindungsgemäßen Geräts.

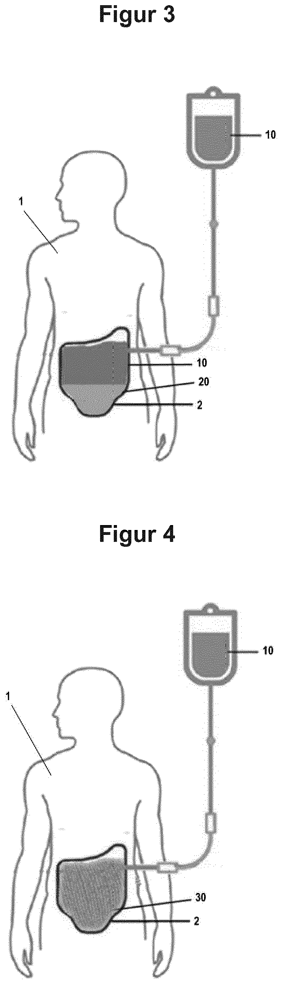

Die Vorteile der Erfindung lassen sich am besten anhand der nachfolgenden Überlegungen erklären. Wie dies aus Figur 3 erkennbar ist, treffen bei jedem neuen Zyklus während des Einlaufs (Inflow) von frischer Dialyselösung 10 im Peritoneum 2 des Patienten 1 vorhandene Residual-Lösung 20 und frische Lösung 10 aufeinander. Die Residual-Lösung 20 wird durch Restflüssigkeit des vorhergehenden Zyklus gebildet und setzt sich aus der zuvor verabreichten Dialysat- und Ultrafiltrationsflüssigkeit zusammen. Die Stoff- Transport-Eigenschaften der Residual-Lösung 20 unterscheiden sich von denjenigen der frischen Lösung 10. Isoliert betrachtet ist es sogar denkbar, dass die Residual-Lösung 20 für ein Resorption sorgt. Die Frage stellt sich wie schnell sich die beiden Lösungen 10 und 20 im Bauchraum miteinander vermischen und welche Eigenschaften diese Mischlösung besitzt. Während des Einlaufes mischt sich ein Teil der frischen Dialysat-Lösung 10 aufgrund der Einströmung mit der Residual-Lösung 20. Die anschließend einsetzende Diffusion bewirkt, dass zu einem Zeitpunkt x ein Konzentrationsausgleich der beiden Lösungen 10 und 20 stattgefunden hat. Vermutlich wird sich nach relativ kurzer Zeitdauer eine homogene Mischlösung 30 im Peritoneum 2 befinden, wie in Figur 4 dargestellt.

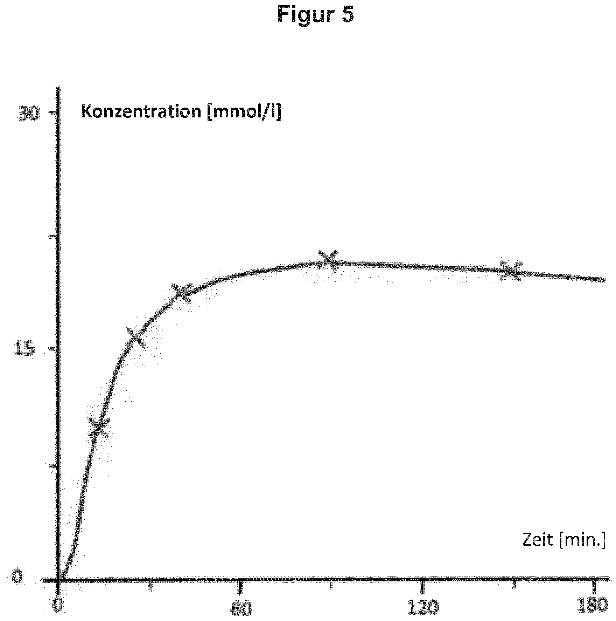

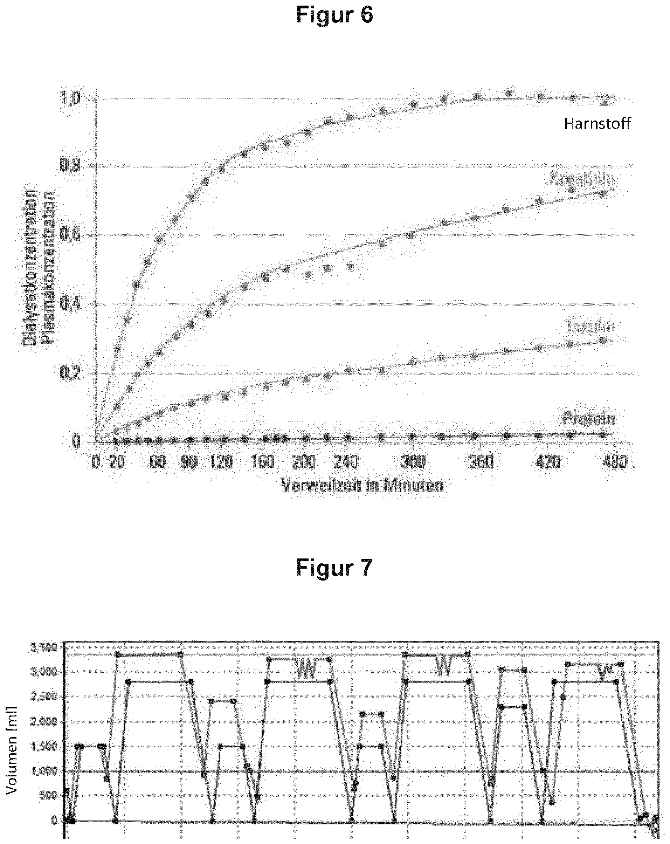

Eine Resorptionskurve, wie beispielhaft in Figur 5 gezeigt, trifft die eine Aussage über die Effektivität der Entgiftung während der Verweilphase (Dwell). Der zeitliche Verlauf der Resorptionskurve ist davon abhängig, ob der Patient ein High- oder Low-Transporter ist. Eine hohe Transportkapazität bedeutet hier, dass schnell ein Konzentrationsgleichgewicht der gelösten Teilchen zwischen Blut und Dialysat erfolgt, entsprechend einem steilen Kurvenverlauf zu Beginn und einem schnellen Erreichen eines Steady State. Weiterhin hängt die Steilheit der Resorptionskurve von der Glukosekonzentration oder der Art der verwendeten Lösung ab. Wie dies der Figur 6 entnommen werden kann, besitzen unterschiedliche Stoffe unterschiedliche Resorptionskurven. Für große Moleküle wie Proteine und Insulin verlaufen diese allgemein flach und annähernd linear.

Gemeinsam ist allen Resorptionskurven für kleinere Moleküle, dass das Konzentrationsgefälle zu Beginn der Verweilphase relativ hoch und linear verläuft. Das bedeutet, dass der effektive Stofftransport in der Anfangszeit konstant bleibt. Die Abhängigkeit des Stofftransportes vom verabreichten Einlaufvolumen ist in Figur 6 nicht dargestellt. Vielmehr wird bei den Darstellungen davon ausgegangen, dass der Patient mit frischer Lösung ideal gefüllt wurde. In der Realität müssten aber auch die Eigenschaften der Residual-Lösung 20 sowie deren Menge berücksichtigt werden. Jedenfalls ist davon auszugehen, dass es durch die Beeinflussung der Residual-Lösung zu einer Verschlechterung der Stoff-Transport-Eigenschaften gegenüber den idealisierten Betrachtungen der Figur 6 kommt.

Gemäß der vorliegenden Erfindung ist nun vorgesehen, nicht benötigtes Einlaufvolumen zu nützen, um während der Verweilphase (Dwell) kleine Teile der angebrauchten Lösung des Peritoneums auszutauschen, um einen zusätzlichen Stofftransport zu erreichen. Dieses Konzept ist im Diagramm der Figur 7 visualisiert, wobei die y-Achse das Füllvolumen im Peritoneum des Patienten zeigt. Im Rahmen des gezeigten Beispiels sind das zulässige Residualvolumen auf 1000 ml (untere horizontale Linie) und das zulässige Patientenvolumen auf 3400 ml (obere horizontale Linie) festgelegt. Die Verschreibung wird durch die theoretische (tieferliegende) Behandlungskurve gezeigt und berücksichtigt kein Residualvolumen. Im Rahmen der tatsächlichen Behandlung gemäß der höherliegenden Behandlungskurve bleibt immer dann eigentlich gemäß Verschreibung eingeplante Dialyseflüssigkeit übrig, wenn das verschriebene Einlaufvolumen gemeinsam mit dem vor Beginn der jeweiligen Einlaufphase noch im Peritoneum vorhandenen Residualvolumen zu einem Gesamtvolumen führen würde, welches das zulässige Patientenvolumen übersteigt. Im gezeigten Beispiel findet in Zyklen 4, 6 und 8 jeweils ein zusätzlicher Teilersatz während der Verweilphase statt, wobei die nicht genutzte und mithin überschüssige Einlauf-Lösung hierbei verbraucht wird. Dies ist durch die gezackten Linien symbolisiert. In den ungeraden Zyklen findet kein Teilersatz statt, weil die Verweilzeiten im Verhältnis geringer sind, sodass der Einsatz des verfügbaren Überschussvolumens in diesen Zyklen weniger effektiv wäre. In Zyklus 2 ist noch kein Residualvolumen verfügbar.

Im Rahmen der Erfindung soll der Teilersatz immer während einer Verweilphase (Dwell) stattfinden. Bevorzugte Zeitpunkte für den Beginn eines Teilersatzes beginnen nach Ablauf von etwa der halben Verweilzeit und enden bei Ablauf von etwa 80% der Verweilzeit, da zu diesem Zeitpunkt im Normalfall das Dialysat nicht mehr so wirksam ist, wie zu Beginn der Behandlung, und andererseits die aufgefrischte Lösung ihre Wirkung noch ausreichend entfalten kann.

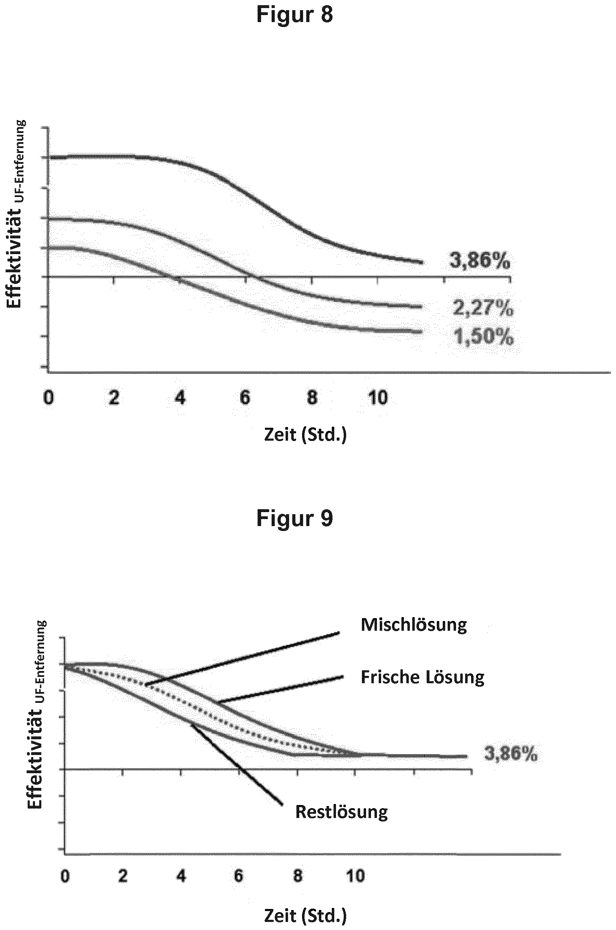

In einem Beispiel kann das verschriebene Einlaufvolumen 3000 ml betragen und das Residualvolumen kann auf 50%, also 1500 ml festgelegt werden. Das Maximalvolumen wird auf 110%, also 3300 ml festgelegt. Wird direkt bei Erreichen des Residualvolumens eine neue Einlaufphase gestartet, dürfen also maximal lediglich 1800 ml in den Patienten einlaufen. Der prozentuale Anteil der Residual-Lösung beträgt somit (1500/3300)*100% = 45%. Da die Effektivität von Dialyselösungen mit der Verweildauer im Peritoneum abnimmt, wie aus dem in Figur 8 dargestellten Zusammenhang ersichtlich, kann dies signifikante Auswirkungen auf die Behandlungseffizienz haben.

Unter der Annahme, dass die Residualflüssigkeit nur aus Dialysat zusammengesetzt ist (kein UF-Anteil), die sich durchschnittlich seit ca. 2 h im Bauchraum befindet ergibt sich für die im Beispiel genannte Konstellation eine Effektivität der Mischlösung, die zwischen der Effektivität insgesamt frischer Dialyselösung und der 45/55-Mischung liegt. Dies ist graphisch in Figur 9 veranschaulicht, wo die Effektivität des Entzugs für UF-Volumens (= UF-Effektivität) für die frische Lösung dargestellt ist. Die UF-Effektivität der Residual-Lösung resultiert aus der um 2 Stunden nach links verschobenen UF-Effektivitätskurve der Frischlösung. Die Effektivität der Mischlösung ergibt sich durch additive Überlagerung unter Rückgriff auf die Annahme, dass das Mischungsverhältnis wie im obigen Rechenbeispiel etwa 50% beträgt. In der Realität wird die Residual-Lösung noch Ultrafiltrationsvolumen enthalten, welche die Effektivität Null besitzt. Durch den erfindungsgemäßen Teilersatz der Lösung wird also erreicht, dass das Blut-Dialysat-Konzentrationsgefälle erhöht wird und somit die Effektivität der Behandlung steigt.

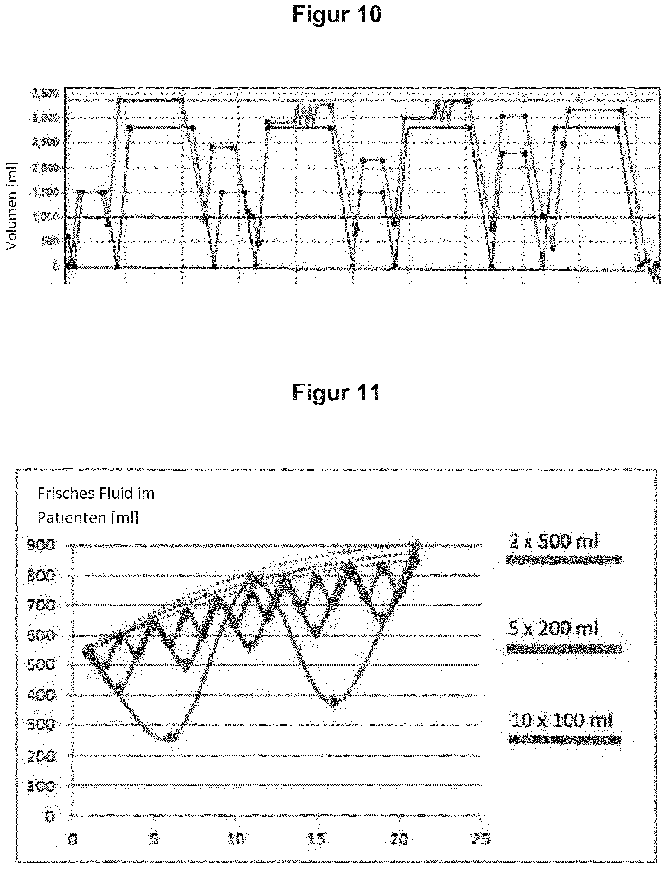

Eine ebenfalls erfindungsgemäße Alternative zu dem in Figur 7 dargestellten Ablauf ist in Figur 10 dargestellt. Dort beginnen die Teilersatzzyklen mit einem Einlauf, um Auslaufdruckprobleme generell zu vermeiden. Das Volumen der vorherigen Einlaufphase kann in dieser Variante um das Volumen des Einlaufs für den Teilersatzzyklus reduziert werden, wie in der Figur erkenntlich gemacht ist.

Der Teilersatz wird in sowohl der Ausführungsform der Figur 7 als auch der Ausführungsform der Figur 10 auf mehrere Zyklen verteilt. Dies hat einerseits Effizienzgründe, andererseits kann sich die Situation bezüglich vorhandenen Restvolumen aber auch im Laufe der Behandlung ändern. So muss beispielsweise gegebenenfalls Dialyselösung verworfen werden, wenn Störungen auftreten oder Pumpen neu positioniert werden.

Im Rahmen der Erfindung kann es sinnvoll sein, das effektive Teilersatzvolumen während des Teilersatzvorgangs so hoch wie möglich zu gestalten. In Figur 11 ist, unter Rückgriff auf das obige Rechenbeispiel, der Anteil der frischen Lösung im Peritoneum in Abhängigkeit von Anzahl und Volumen der Teilersatzzyklen dargestellt. Nachdem der reguläre Einlauf verabreicht wurde befanden sich 550 ml frische Lösung und 450 ml Residuallösung im Bauchraum des Patienten. Je höher die Austauschvolumina, desto höher ist am Ende der Anteil an frischer Lösung, wie sich auch aus der nachfolgenden Tabelle 1 entnehmen lässt, welche die Werte des Diagramms der Figur 11 wiederspiegelt.

**Tabelle 1**

| Teilersatzzyklen | Frische Lösung im Bauchraum vorher | Frische Lösung im Bauchraum nachher |
|---|---|---|
| 10 × 100 ml | 550 ml | 843 ml |
| 5 x 200 ml | 550 ml | 852 ml |
| 2 x 500 ml | 550 ml | 887 ml |

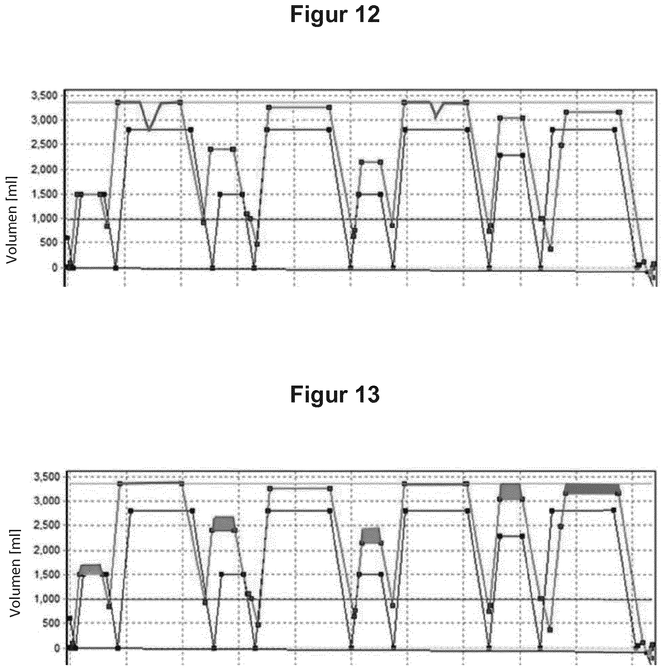

Eine weitere Ausführungsform der Erfindung ist in Figur 12 dargestellt. In dieser Ausführungsform werden lediglich im Rahmen zweier Zyklen Teilersatzvorgänge vorgenommen. Für Zyklen mit kurzen Verweilzeiten macht es auf Grund der kurzen Einwirkzeit gegebenenfalls keinen Sinn einen Teilersatzzyklus zu generieren.

In einer Variante des erfindungsgemäßen Konzepts ist vorgesehen, dass nicht genutzte Einlauf-Lösung alternativ oder zusätzlich verwendet wird, um Einläufe geringfügig zu erhöhen, wie in der Darstellung der Figur 13 ersichtlich. Das maximal zulässige Patenvolumen darf dabei natürlich nicht überschritten werden. Diese Variante der Verfahrensführung könnte für adaptierte Verschreibungen vorteilhaft sein, da sich in dieser Behandlungsform Zyklen mit kleinen und großen Einlaufvolumina abwechseln. Die Zyklen mit den kleinen Einlaufvolumina könnten innerhalb vorgegebener Grenzen von beispielsweise 10% angehoben werden.

Vorteile des erfindungsgemäßen Konzepts umfassen, dass kaum oder zumindest weniger Flüssigkeit in die Drainage geleitet werden. Das gesamte angeschlossene Lösungsvolumen kann für die Behandlung sinnvoll aufgebraucht werden. Zeitaufwändige und know-how-intensive Workarounds wie die Erstellung von Tidal-Verschreibungen oder nachträgliches Editieren werden nicht mehr benötigt. Die Nachteile der Workarounds werden kompensiert. Die Anpassung erfolgt sehr dynamisch und situationsabhängig. Die Effizienz der Behandlung wird erhöht. Der Patient kann nicht überfüllt werden. Es sind keine zeitlichen Verzögerungen der Behandlungen notwendig. Zusätzliche Störungen sind nicht zu erwarten, sodass der Patient wegen der Verfahrensführung nicht geweckt werden muss. Das Konzept funktioniert mit profilierten Behandlungen.

## Patentansprüche

1. Vorrichtung zur Durchführung einer Peritonealdialyse-Behandlung, umfassend ein Reservoir für frische Dialyseflüssigkeit, einen Anschluss zur Verbindung mit einem Peritoneal-Katheter eines Patienten, einen Ablauf für verbrauchte Dialyseflüssigkeit und eine mit Aktoren in Verbindung stehende Steuereinheit, wobei die Vorrichtung ausgebildet ist, auf der Grundlage einer in der Steuereinheit hinterlegten Verschreibung für eine Behandlung mehrere aufeinanderfolgende Inflow-Dwell-Outflow-Zyklen durchzuführen, in welchen dem Patienten Dialyseflüssigkeit aus dem Reservoir zugeführt und nach Ablauf einer bestimmten Verweilzeit im Peritoneum des Patienten wieder durch den Ablauf abgeleitet wird,
**dadurch gekennzeichnet,**
**dass** die Vorrichtung ferner ausgebildet ist, in wenigstens einem Zyklus einen Teil der im Peritoneum des Patienten befindlichen Dialyseflüssigkeit bereits vor Ablauf der Verweilzeit mit frischer Dialyseflüssigkeit aus dem Reservoir zu ersetzen, wenn in wenigstens einem vorhergehenden Zyklus das gemäß Verschreibung für diesen vorhergehenden Zyklus vorgesehene Volumen an frischer Dialyseflüssigkeit nicht vollständig verbraucht wurde.

2. Vorrichtung nach Anspruch 1, **dadurch gekennzeichnet, dass** im Rahmen des vorzeitigen Teilersatzes maximal 50%, vorzugsweise maximal 30% und weiter vorzugsweise maximal 20% des insgesamt im Peritoneum befindlichen Volumens an Dialyseflüssigkeit ersetzt werden.

3. Vorrichtung nach einem der vorhergehenden Ansprüche, **dadurch gekennzeichnet, dass** die Verweilzeit durch den vorzeitigen Teilersatz nicht verlängert oder verkürzt wird und/oder dass das im Peritoneum des Patienten befindliche Gesamtvolumen an Dialyseflüssigkeit durch den vorzeitigen Teilersatz nicht verändert wird.

4. Vorrichtung nach einem der vorhergehenden Ansprüche, **dadurch gekennzeichnet, dass** der vorzeitige Teilersatz in mehreren vorzugsweise unmittelbar aufeinanderfolgenden Zu- und Ablaufschritten durchgeführt wird, wobei vorzugsweise wenigstens fünf Zulaufschritte vorgesehen sind und/oder mit jedem Zulaufschritt maximal 200 ml zugeführt werden.

5. Vorrichtung nach einem der vorhergehenden Ansprüche, **dadurch gekennzeichnet, dass** der vorzeitige Ersatz erst nach Ablauf von wenigstens 50% der vorgesehenen Verweilzeit durchgeführt wird und/oder dass der vorzeitige Ersatz vor Ablauf von maximal 80% der vorgesehenen Verweilzeit beendet ist.

6. Vorrichtung nach einem der vorhergehenden Ansprüche, **dadurch gekennzeichnet, dass** die Vorrichtung ausgebildet ist, über den Verlauf der Behandlung in mehreren Zyklen einen Teil der im Peritoneum des Patienten befindlichen Dialyseflüssigkeit bereits vor Ablauf der Verweilzeit mit frischer Dialyseflüssigkeit aus dem Reservoir zu ersetzen.

7. Vorrichtung nach einem der vorhergehenden Ansprüche, **dadurch gekennzeichnet, dass** die Vorrichtung ausgebildet ist, einen Teilersatzzyklus nur dann zu starten, wenn die gemäß Verschreibung für den jeweiligen Zyklus vorgesehene Verweilzeit eine Mindestzeit überschreitet.

8. Vorrichtung zur Durchführung einer Peritonealdialyse-Behandlung, umfassend ein Reservoir für frische Dialyseflüssigkeit, einen Anschluss zur Verbindung mit einem Peritoneal-Katheter eines Patienten, einen Ablauf für verbrauchte Dialyseflüssigkeit und eine mit Aktoren in Verbindung stehende Steuereinheit, wobei die Vorrichtung ausgebildet ist, auf der Grundlage einer in der Steuereinheit hinterlegten Verschreibung für eine Behandlung mehrere aufeinanderfolgende Zyklen durchzuführen, in welchen dem Patienten Dialyseflüssigkeit aus dem Reservoir zugeführt und nach Ablauf einer bestimmten Verweilzeit im Peritoneum des Patienten wieder durch den Ablauf abgeleitet wird, insbesondere Vorrichtung nach einem der vorhergehenden Ansprüche,
**dadurch gekennzeichnet,**
**dass** die Vorrichtung ferner ausgebildet ist, in wenigstens einem Zyklus das gesamte Einlaufvolumen zu erhöhen, wenn in wenigstens einem vorhergehenden Zyklus das gemäß Verschreibung für diesen vorhergehenden Zyklus vorgesehene Volumen an frischer Dialyseflüssigkeit nicht vollständig verbraucht wurde.

9. Vorrichtung nach einem der vorhergehenden Ansprüche, **dadurch gekennzeichnet, dass** in der Steuereinheit ein Maximalvolumen an Dialyseflüssigkeit festgelegt ist, das sich im Peritoneum des Patienten befinden darf, und dass der nicht vollständige Verbrauch des für den vorhergehenden Zyklus vorgesehenen Volumens an frischer Dialyseflüssigkeit daraus resultiert, dass der vollständige Verbrauch zu einer Überschreitung des Maximalvolumens geführt hätte.

10. Vorrichtung nach einem der vorhergehenden Ansprüche, **dadurch gekennzeichnet, dass** die Vorrichtung ausgebildet ist, dem Patienten im Verlauf der Behandlung die gemäß der Verschreibung vorgesehene Gesamtmenge der Dialyseflüssigkeit zuzuführen, wobei eine etwaige Differenzmenge, die nicht im Rahmen der regulären Einlaufphasen zugeführt wird, insbesondere aufgrund der Berücksichtigung des Maximalvolumens, vollständig im Rahmen des vorzeitigen Ersatzes während des wenigstens einen oder mehrerer Zyklen zugeführt wird.

## Claims

1. Apparatus for carrying out a peritoneal dialysis treatment comprising a reservoir for fresh dialysis fluid, a connector for connecting to a peritoneal catheter of a patient, a drain for consumed dialysis fluid, and a control unit connected to actuators, wherein the apparatus is configured to carry out a plurality of consecutive inflow-dwell-outflow cycles for a treatment on the basis of a prescription stored in the control unit, in which inflow-dwell-outflow cycles dialysis fluid is supplied to the patient from the reservoir and is led off through the outflow again after the elapse of a specific dwell time in the peritoneum of the patient,
**characterized in that**
the apparatus is further configured to already replace a portion of the dialysis fluid present in the peritoneum of the patient with fresh dialysis fluid from the reservoir before the elapse of the dwell time in at least one cycle if, in at least one preceding cycle, the volume of fresh dialysis fluid provided in accordance with the prescription for this preceding cycle was not completely consumed.

2. Apparatus in accordance with claim 1, **characterized in that** a maximum of 50%, preferably a maximum of 30%, and further preferably a maximum of 20%, of the total volume of dialysis fluid present in the peritoneum is replaced as part of the premature partial replacement.

3. Apparatus in accordance with one of the preceding claims, **characterized in that** the dwell time is not extended or reduced by the premature partial replacement and/or **in that** the total volume of dialysis fluid present in the peritoneum of the patient is not changed by the premature partial replacement.

4. Apparatus in accordance with one of the preceding claims, **characterized in that** the premature partial replacement is carried out in a plurality of inflow and removal steps, preferably directly consecutive, with at least five inflow steps preferably being provided and/or with a maximum of 200 ml being supplied with each inflow step.

5. Apparatus in accordance with one of the preceding claims, **characterized in that** the premature replacement is only carried out after the elapse of at least 50% of the provided dwell time and/or that the premature replacement is ended before the elapse of a maximum of 80% of the provided dwell time.

6. Apparatus in accordance with one of the preceding claims, **characterized in that** the apparatus is configured to already replace a portion of the dialysis fluid present in the peritoneum of the patient in a plurality of cycles over the course of the treatment with fresh dialysis fluid from the reservoir before the elapse of the dwell time.

7. Apparatus in accordance with one of the preceding claims, **characterized in that** the apparatus is configured only to start a partial replacement cycle when the dwell time provided for the respective cycle in accordance with the prescription exceeds a minimum time.

8. Apparatus for carrying out a peritoneal dialysis treatment comprising a reservoir for fresh dialysis fluid, a connector for connecting to a peritoneal catheter of a patient, a drain for consumed dialysis fluid, and a control unit connected to actuators, wherein the apparatus is configured to carry out a plurality of consecutive cycles for one treatment on the basis of a prescription stored in the control unit, in which inflow-dwell-outflow cycles dialysis fluid is supplied to the patient from the reservoir and is led off through the drain again after the elapse of a specific dwell time in the peritoneum of the patient, in particular an apparatus in accordance with one of the preceding claims,
**characterized in that**
the apparatus is further configured to increase the total inflow volume in at least one cycle if, in at least one preceding cycle, the volume of fresh dialysis fluid provided in accordance with the prescription for this preceding cycle was not completely consumed.

9. Apparatus in accordance with one of the preceding claims, **characterized in that** a maximum volume of dialysis fluid that may be present in the peritoneum of the patient is fixed in the control unit and that the incomplete consumption of the volume of fresh dialysis solution provided for the preceding cycle results from the fact that the complete consumption would have led to an exceeding of the maximum volume.

10. Apparatus in accordance with one of the preceding claims, **characterized in that** the apparatus is configured to supply the patient with the total volume of dialysis fluid provided in accordance with the prescription in the course of the treatment, with any difference quantity that is not supplied as part of the regular inflow phases being completely supplied, in particular on the basis of a consideration of the maximum volume, as part of the premature replacement during the at least one or more cycles.

## Revendications

1. Dispositif pour l'exécution d'un traitement de dialyse péritonéale, comprenant un réservoir pour du liquide de dialyse frais, un raccord pour la connexion à un cathéter péritonéal d'un patient, une évacuation pour le liquide de dialyse utilisé et une unité de commande en liaison avec des actionneurs, le dispositif étant configuré pour exécuter, sur la base d'une prescription pour un traitement enregistrée dans l'unité de commande, plusieurs cycles successifs de remplissage-pause-vidange, dans lesquels le liquide de dialyse est amené au patient à partir du réservoir et est à nouveau déchargé par l'évacuation après l'écoulement d'un temps de séjour déterminé dans le péritoine du patient,
**caractérisé en ce que**
le dispositif est en outre configuré pour remplacer déjà, avant l'écoulement du temps de séjour, dans au moins un cycle, une partie du liquide de dialyse se trouvant dans le péritoine du patient par du liquide de dialyse frais provenant du réservoir si, dans au moins un cycle précédent, le volume de liquide de dialyse frais prévu pour ce cycle précédent conformément à la prescription n'a pas été entièrement utilisé.

2. Dispositif selon la revendication 1, **caractérisé en ce que**, dans le cadre du remplacement partiel anticipé, on remplace au maximum 50 %, de préférence au maximum 30 % et de préférence encore au maximum 20 % du volume total de liquide de dialyse se trouvant dans le péritoine.

3. Dispositif selon l'une quelconque des revendications précédentes, **caractérisé en ce que** le temps de séjour n'est pas prolongé ou raccourci par le remplacement partiel anticipé et/ou **en ce que** le volume total de liquide de dialyse se trouvant dans le péritoine du patient n'est pas modifié par le remplacement partiel anticipé.

4. Dispositif selon l'une quelconque des revendications précédentes, **caractérisé en ce que** le remplacement partiel anticipé est exécuté en plusieurs étapes d'alimentation et d'évacuation qui se succèdent de préférence immédiatement, au moins cinq étapes d'alimentation étant de préférence prévues et/ou un maximum de 200 ml étant amené à chaque étape d'alimentation.

5. Dispositif selon l'une quelconque des revendications précédentes, **caractérisé en ce que** le remplacement anticipé n'est exécuté qu'après l'écoulement d'au moins 50 % du temps de séjour prévu et/ou **en ce que** le remplacement anticipé est terminé avant l'écoulement d'au maximum 80 % du temps de séjour prévu.

6. Dispositif selon l'une quelconque des revendications précédentes, **caractérisé en ce que** le dispositif est configuré pour remplacer déjà, avant l'écoulement du temps de séjour, en plusieurs cycles, au cours du traitement, une partie du liquide de dialyse se trouvant dans le péritoine du patient par du liquide de dialyse frais provenant du réservoir.

7. Dispositif selon l'une quelconque des revendications précédentes, **caractérisé en ce que** le dispositif est configuré pour ne démarrer un cycle de remplacement partiel que si le temps de séjour prévu pour chaque cycle conformément à la prescription dépasse un temps minimum.

8. Dispositif pour l'exécution d'un traitement de dialyse péritonéale, comprenant un réservoir pour du liquide de dialyse frais, un raccord pour la connexion à un cathéter péritonéal d'un patient, une évacuation pour le liquide de dialyse utilisé et une unité de commande en liaison avec des actionneurs, le dispositif étant configuré pour exécuter, sur la base d'une prescription pour un traitement enregistrée dans l'unité de commande, plusieurs cycles successifs au cours desquels du liquide de dialyse est amené au patient à partir du réservoir et est à nouveau déchargé par l'évacuation après l'écoulement d'un temps de séjour déterminé dans le péritoine du patient, notamment dispositif selon l'une quelconque des revendications précédentes,
**caractérisé en ce que**
le dispositif est en outre configuré pour augmenter le volume d'entrée total dans au moins un cycle si, dans au moins un cycle précédent, le volume de liquide de dialyse frais prévu conformément à la prescription pour ce cycle précédent n'a pas été entièrement utilisé.

9. Dispositif selon l'une quelconque des revendications précédentes, **caractérisé en ce qu'**un volume maximal de liquide de dialyse pouvant se trouver dans le péritoine du patient est établi dans l'unité de commande et **en ce que** l'utilisation incomplète du volume de liquide de dialyse frais prévu pour le cycle précédent résulte du fait que l'utilisation complète aurait conduit à un dépassement du volume maximal.

10. Dispositif selon l'une quelconque des revendications précédentes, **caractérisé en ce que** le dispositif est configuré pour amener au patient, au cours du traitement, la quantité totale de liquide de dialyse prévue conformément à la prescription, une éventuelle quantité différentielle non amenée dans le cadre des phases d'entrée régulières, notamment en raison de la prise en compte du volume maximal, étant amenée en totalité dans le cadre du remplacement anticipé pendant l'au moins un ou plusieurs cycles.
